(19) **Europäisches Patentamt**

**European Patent Office**

**Office européen des brevets**

(11) **EP 1 342 782 A1**

(12) **EUROPEAN PATENT APPLICATION**
published in accordance with Art. 158(3) EPC

(43) Date of publication:
**10.09.2003 Bulletin 2003/37**

(51) Int Cl.⁷: **C12N 15/00**, G06F 19/00, C12M 1/00

(21) Application number: **01981092.8**

(22) Date of filing: **13.11.2001**

(86) International application number:
**PCT/JP01/09909**

(87) International publication number:
**WO 02/038749 (16.05.2002 Gazette 2002/20)**

(84) Designated Contracting States:
**AT BE CH CY DE DK ES FI FR GB GR IE IT LI LU MC NL PT SE TR**

(30) Priority: **13.11.2000 JP 2000345982**

(71) Applicant: **JAPAN SCIENCE AND TECHNOLOGY CORPORATION**
**Kawaguchi-shi, Saitama 332-0012 (JP)**

(72) Inventors:
- **KYODA, Koji**
  **Yokohama-shi, Kanagawa 227-0036 (JP)**
- **ONAMI, Shuichi**
  **Setagaya-ku, Tokyo 158-0082 (JP)**

(74) Representative: **Woods, Geoffrey Corlett**
**J.A. KEMP & CO.,**
**Gray's Inn,**
**14 South Square**
**London WC1R 5JJ (GB)**

(54) **METHOD OF ANTICIPATING GENE NETWORK, ANTICIPATION SYSTEM AND RECORDING MEDIUM**

(57) A method of predicting a gene network which comprises detecting, when an expression amount of a single gene among a plurality of genes is expressed under 2 conditions, the respective expression amounts of another gene, determining a difference between the detected values, and deriving a causal relationship between the single gene and the other gene using the difference as an indicator.

Fig.2

**Description**

Technical Field

The present invention relates to a method of predicting a gene network, a prediction system and a recording medium.

Background Art

Due to recent advances in the field of molecular biology, an enormous amount of gene information is now available. As a consequence it is necessary to make efforts using computers to extract information from the rapidly increasing number of sequence data, and gene expression data that are successively being clarified. To the present day, development of various computer tools such as homology screening, protein classification and gene pooling, etc. have been attracting attention.

Among such attempts, there are a number, though only several, examples of research concerning methods of inferring a gene regulation network from gene expression data. Gene expression data can be obtained in either the form of time series data ("time series data" refers to data obtained by measurement of gene expression amounts of a subject group of genes over the course of time), or steady state data ("steady state data" refers to data obtained by measurement of gene expression amounts of a subject group of genes under a plurality of differing experimental conditions (for example, gene mutation or administration of a medicament)).

In the case of a method of analyzing a time series, a network can be predicted using various methods, for example, information theory, heredity algorithm or simulated annealing (Liang, S. et al., Proc. Pacific Symp. Biocomputing '98, World Scientific, 18-29, 1998.; Morohashi, M. and Kitano, H., Proc. 5th Euro. Conf. Artificial Life, Springer, 477-486, 1999.; Mjolsness, E., et al., Tech. Rept. JPL-ICTR-99-4, Jet Propulsion Lab., NASA, 1999.). However, a time series approach requires that experimental results are obtained at extremely short intervals and in a form free of experimental noise. This is extremely difficult with current techniques.

On the other hand, a number of methods of analysis of a steady state have already been advanced.

This steady state data can be obtained by mutating a specific gene activity, for example by causing deletion or over-expression of a gene. Deletion is presently being performed on a large scale by the Yeast Genome Deletion Consortium, etc., and as a result, deletion-type expression profiles for various genes will become readily available in the near future. (Winzeler, E. A. et al., Science, 285 (5429): 901-906, 1999.)

Akutsu, et al. calculated the upper and lower limits for the number of experiments required where a gene network is expressed with a Boolean network model, (i.e. where the gene expression of each gene is expressed in two states: active or inactive; and the relationship between these genes is expressed with Boolean algebra (Akutsu, T. et al., Proc. 9th ACM-SIAM Symp. Discrete Algorithms, 695-702, 1998.). Further, recently Ideker, et al. advanced a method known as the Predictor method (Ideker, T. E. et al., Proc. Pacific Symp. Biocomputing 2000, World Scientific, 305-316, 2000.) This method provides candidate networks (Boolean network models matching expression data) using an optimization method relating to combinations.

However, these methods express gene expression levels as a gene network in binary. Consequently, although experimental data ordinarily possesses a continuous value (multi-value), in order to apply this method, correction of the data into binary is required. By such a correction, it is possible that information necessary for prediction of a network is lost. For example, by prediction using binary, it is difficult to accurately represent gene expression levels in three states (for example, wild type, deletion, over-expression), resulting in inaccurate reflection of regulatory relationships.

Disclosure of the Invention

The present invention is directed to providing a method of predicting a gene network, a prediction system and a recording medium.

The present inventors, as a result of deliberate research to solve the above problem, succeeded in performing prediction of a gene network using a gene expression profile (detected value) obtained when mutation(s) were induced, without simplification (binarization) of gene expression amounts, thereby completing the present invention.

That is, the present invention is a method of predicting a gene network which comprises detecting, when an expression amount of a single gene among a plurality of genes is expressed under 2 conditions, the respective expression amounts of another gene; determining a difference between the obtained detected values; and deriving a causal relationship between the single gene and the other gene using the difference as an indicator.

Further, the present invention is a method of predicting a gene network which comprises respectively detecting, when a single gene among a plurality of genes has been mutated, an expression level of another gene, and when the single gene has not been mutated, an expression level of the other gene; determining the difference between the obtained detected values; and deriving a causal relationship between the single gene and the other gene using the difference as an indicator. Examples of a gene network include one within a single-celled organism or one within a multi-cellular organism. Examples of a gene mutation include gene disruption and over-expression. In the above-described prediction method, it is preferable to verify the presence or absence of a contradiction in the obtained causal relationship.

Further, the present invention is a gene network prediction system comprising a means for detecting, when an expression amount of a single gene among a plurality of genes is expressed under 2 conditions, the respective expression amounts of another gene; a means for determining a difference between the detected values; and a means for deriving a causal relationship between the single gene and the other gene using the difference as an indicator.

Further, the present invention is a gene network prediction system comprising a means for detecting, when a single gene among a plurality of genes has been mutated, an expression level of another gene, and when the single gene has not been mutated, an expression level of the other gene; a means for determining a difference between the detected values; and a means for deriving a causal relationship between the single gene and the other gene using the difference as an indicator. A means for verifying the presence or absence of a contradiction in an obtained causal relationship can be included in the prediction system according to the present invention.

Further, the present invention is a computer readable recording medium on which there is recorded a program causing a computer to function as a gene network prediction system, which comprises a means for detecting, when a single gene among a plurality of genes has been mutated, an expression level of another gene, and when the single gene has not been mutated, an expression level of the other gene; a means for determining a difference between the detected values; and a means for deriving a causal relationship between the single gene and the other gene using the difference as an indicator.

Below, the present invention is explained in detail. This description encompasses the content described in the specification and drawings of Japanese Patent Application No. 2000-345982 which forms a basis of the priority claim of the present application.

The present invention focuses on how, when an expression amount of a single gene (gene a) among a plurality of genes, is expressed respectively under 2 conditions (condition 1 and condition 2), the expression amount of another gene (gene b) changes as between condition 1 and condition 2, and is characterized by examining (detecting) the respective expression amounts of gene b under each condition, determining a difference between the examined values (detected values), and deriving a causal relationship between the single gene a and the other gene b using the difference as a indicator.

For example, the case of the wild type of gene a (condition 1), and the case of a mutated gene a (condition 2) are established as the 2 conditions. A causal relationship between gene a and gene b is determined by detecting the difference in expression amounts of the other gene b under these 2 conditions, and the gene network arising between these genes, is predicted.

The present invention is characterized in that it treats all changing values as continuous values (multi-values) rather than as binary values such as ON and OFF relationships. Consequently, a network can be predicted with higher precision than by binary.

1. Gene Network Prediction Method and Prediction System

The prediction system of the present invention is constituted by a device comprising:

(i) means for detecting, where 2 conditions are established for an expression amount of a single gene, the respective expression levels (expression amounts) of the other gene (also referred to as a "detection engine");
(ii) means for comparing and determining the difference between the obtained detected values (also referred to as a "comparison engine");
(iii) means for determining a causal relationship between the single gene and the other gene (also referred to as a "causal relationship creation engine") using the difference as an indicator; and
(iv) means for deleting a redundant causal relationship from among the causal relationships (also referred to as a "redundant relationship deletion engine").

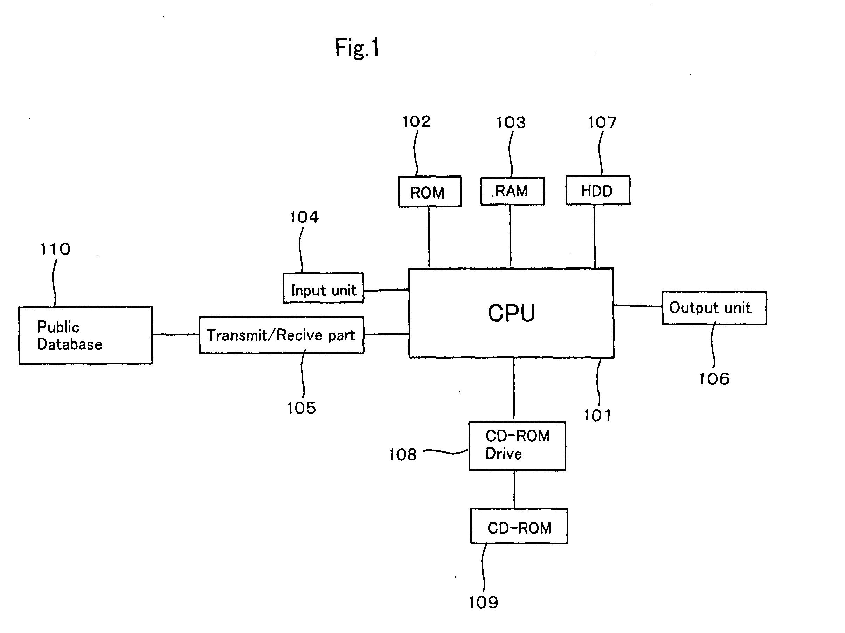

Here, an example of the constitution of the prediction system of the present invention is shown as a block diagram (Fig. 1).

The prediction system shown in Fig. 1 is provided with a CPU101, ROM102, RAM103, input unit 104, transmit/receive part 105, output unit 106, hard disk drive (HDD) 107 and CD-ROM drive 108.

In accordance with a program stored in ROM102, RAM103 or HDD107, CPU101 controls the entire gene network system and performs the below-described network prediction processing. ROM102 stores programs, etc. which instruct the necessary processing for operation of the gene network prediction system. RAM103 temporarily stores data required for execution of network prediction processing. Input unit 104 is a keyboard, mouse or the like, and is operated when inputting necessary conditions for execution of network prediction processing, etc. Transmit/receive part 105, based on instructions from CPU 101, executes transmit/receive processing of data with public database 110, etc, via a telecommunications circuit. Output unit 106 executes display processing, based on instructions from CPU 101, of gene expression amounts, various conditions input from input unit 104, gene nucleotide sequences, and results of network prediction, etc. Examples of output unit 106 include computer displays, or printers, etc. HDD107 stores a gene network prediction program, gene expression amounts, and nucleotide sequences, etc., and on the basis of instructions of CPU101, stored programs or data etc., are read and stored, for example, in RAM103. CD-ROM drive 108, based on instructions from CPU 101, reads programs or data from gene network prediction programs or expression amounts, etc. stored on CD-ROM 109, and stores them in, for example, RAM 103.

CPU101 supplies data received from input units, etc. to output unit 106, and at the same time executes gene network prediction processing based on data received from a database.

Here, the processing program for predicting a gene network, as described below, is constituted by an engine for detecting gene expression amounts, a comparison engine and an engine for creating causal relationships.

(1) Detection Engine for Gene Expression Amount

Detection of gene expression in the present invention can be performed by use or application of DNA microarray, oligonucleotide chip, RT-PCR, continuous analysis of gene expression, steady state expression level, proteomics, etc., and results therefrom can be used as gene expression data (expression amount). Further, all state change that regulate gene function expression, such as protein phosphorylation state or chromosomal DNA methylation state, etc. can be detected as gene expression. In the present invention, the above data is referred to as a detected value. A detected value may be an absolute value or a relative value. Further, values may be obtained from not only experimentally obtained data, but from literature or gene databases, etc. For example, in respect of expression amounts obtained from a DNA microarray, intensity of fluorescence emanating from the microarray can be measured, and this fluorescence intensity can be converted to numerical values.

Specific examples of a database include a databases usable via the int ernet, specifically, GenBank, DDBJ, EMBL, NCI60, etc.

(2) Comparison Engine

In the comparison engine, in the case where 2 conditions are established for the gene expression amount of a particular gene, information concerning a difference in expression level of the subject gene under these two conditions, and information concerning a function between the above genes (for example, activation or inhibition), is accumulated. Establishment of the 2 conditions for gene expression amount includes gene mutation, gene mutation refers to loss of function of the original gene due to deletion, substitution or addition in the nucleotide sequence, and mutation includes disruption of a gene.

A mutation such as a deletion, substitution or addition can be introduced into a gene, by known methods such as Kunkel method, Gapped duplex method or the like, or by adoption of a method adapted therefrom. Introduction of mutations can be performed, for example, by using a kit for introduction of a mutation using site-specific mutagenesis (for example Mutan-K, (manufactured by TAKARA) or Mutan-G (manufactured by TAKARA)) or TAKARA's LA PCR in vitro Mutagenesis series kit, or the like, or by cleaving a part or all of a gene. Further, expression amount data for when a gene is mutated can be obtained from a database.

(3) Causal Relationship Creation Engine

A causal relationship creation engine is a means for deriving a causal relationship between genes based on a difference in expression amount obtained by the comparison engine, and finally predicting a network. This means, for example, in the case where a causal relationship between gene a and gene b is examined, and when the expression amount of gene b when gene a has been mutated, and the expression amount of gene b when gene a has not been mutated (when it is the wild type), are compared to determine the extent of any change, is a means which executes a judgement as to whether this gene a activates or inhibits, or is unrelated to, gene b.

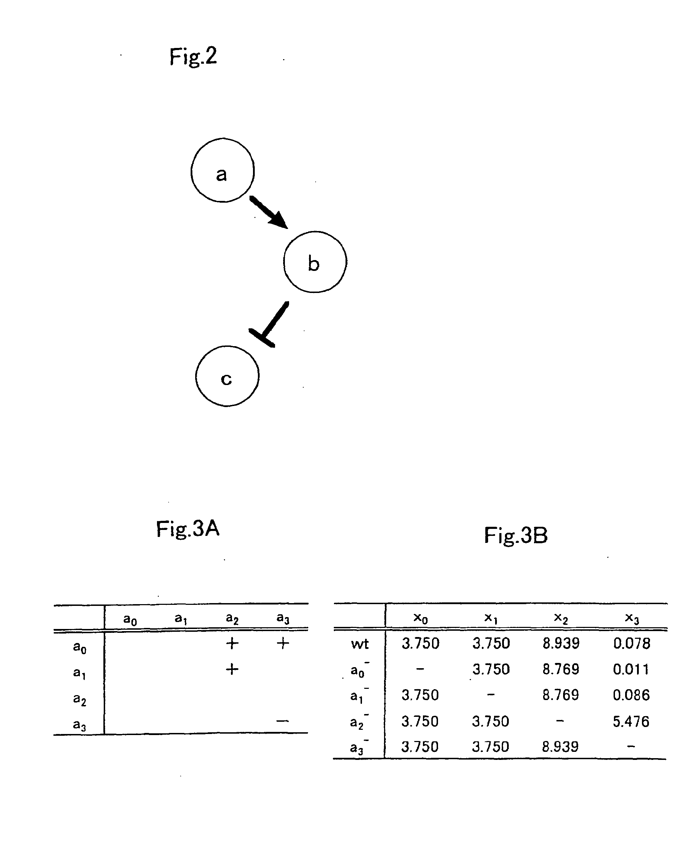

In the present invention, for the sake of convenience, a gene network is considered in terms of a graph known as a directed graph (Figure 2). In figure 2, letters of the alphabet represent genes, an arrow ("→") represents control by gene a which promotes expression of gene b, a T-shaped arrow ("⊣") represents control by gene b which suppresses expression of gene c. In the present invention, an individual arrow is referred to as an "edge". Where a network (causal relationship) between gene a and gene b is predicted, first, 2 conditions are established for an expression amount of gene a, and the expression amount of gene b under these 2 conditions, for example, the expression amount of gene b detected in a steady state or wild type of gene a, where it has not been operated on in any way, and the expression amount of gene b when gene a has been mutated (e.g., disrupted), is detected. Next, the difference the respectively obtained data (detected values) is determined. A causal relationship between the genes is derived using this difference as an indicator. For example, if there is no change in the difference, it can be determined that gene a is not activating gene b, or there is no relationship. If these is a specific change in this difference, it can be determined that gene a activates or inhibits gene b. Here, "a specific change" refers to the exhibition of a change exceed what is known as a threshold value. The threshold value can be established as appropriate depending on the target gene at the time of predicting a network. However, it is preferable to establish and adopt a threshold value with a standard obtained by error-testing, for example, where the significant difference risk becomes 0.01 or less.

Because expression amounts of a gene can generally often be obtained as a numerical value, the interrelationship between gene can be expressed tentatively in the manner shown in Fig. 3. In Fig. 3, $a_n$ (n=0,1,2,3) is called a "node", and refers to an intersection between an edge and another edge created when a gene network is predicted. Because a node can be represented by the name of a gene, the number of nodes and the number of genes (the number n) is identical. $x_n$ (n=0,1,2,3) refers to the expression amount of the corresponding node (gene). wt means wild type.

Results for expression amounts of a particular gene $a_0$ - $a_3$ are obtained as in Fig. 3B. $a_n^-$ (n=0,1,2,3) represents that the gene at each node has been mutated (disrupted). Here, the case where gene $a_0$ has been disrupted ($a_0^-$) is considered.

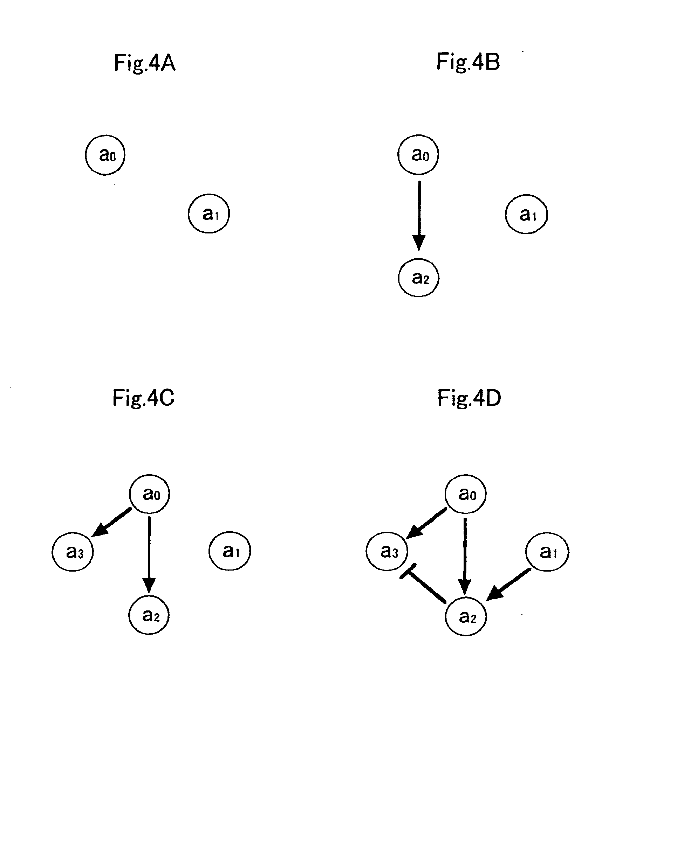

First, because the difference in expression amount of gene $a_1$ for $a_0^-$ and the expression amount of $a_1$ gene for the wild type exhibits no change (3.750-3.750=0), $a_0$ has no relationship to $a_1$, i.e., it can be predicted that there is no network from $a_0$ to $a_1$ and there is no edge between the nodes (Fig. 4A). Next, the difference between the expression amount of gene $a_2$ for $a_0^-$ and the expression amount of gene $a_2$ for the wild type is -0.17 (8.769-8.939=-0.17). That is, if gene $a_0$ is disrupted, the expression amount of $a_2$ decreases. Therefore, if the threshold value is ignored, then it can be predicted that gene $a_0$ activates $a_2$, and an edge can be drawn from node $a_0$ leading to $a_2$ (that is, an arrow is indicated)(Fig. 4B). Further, the difference between the expression amount of gene $a_3$ for $a_0^-$ and the expression amount of gene $a_3$ for the wild type is -0.067 (0.011-0.078=-0.067). That is, if gene $a_0$ is disrupted, the expression amount of $a_3$ decreases. Therefore, gene $a_0$ can be predicted to be a gene which activates $a_3$, and an edge can be drawn from $a_0$ leading to $a_3$. (Fig. 4C).

By processing the cases where gene $a_1$ is disrupted, gene $a_2$ is disrupted, and gene $a_3$ is disrupted, in the same general manner as described above, a network between the 4 genes can be predicted (Fig. 4D). In particular, the difference between the expression amount of gene $a_3$ where gene $a_2$ was disrupted and the expression amount of gene $a_3$ for the wild type was 5.398 (5.476-0.078=5.398). That is, if gene $a_2$ is disrupted, the expression amount of $a_3$ increases. Therefore, it can be predicted that gene $a_2$ represses gene $a_3$, and the edge between the nodes becomes an arrow indicating inhibition (T-shaped arrow)(Fig. 4D).

The relationships of the above-described network are brought together in Fig. 3A. In Fig. 3A, "+" indicates activation (→), and "-" indicates inhibition (⊣).

The relationship between an increase and decrease of expression level of gene b when a gene a is deleted or over-expressed, is shown in Table 1. The quantity of calculations for this comparison process is $O(n^2)$.

Table 1 -

| Relationship to Rise or Fall in Expression Level of a Gene | | | |
|---|---|---|---|
| | | Gene b Expression Level | |
| | | Increase | Decrease |
| Gene a | Deletion | a ⊣ b | a → b |
| | Over-expression | a → b | a ⊣ b |

(4) Redundant Relationship Deletion Engine

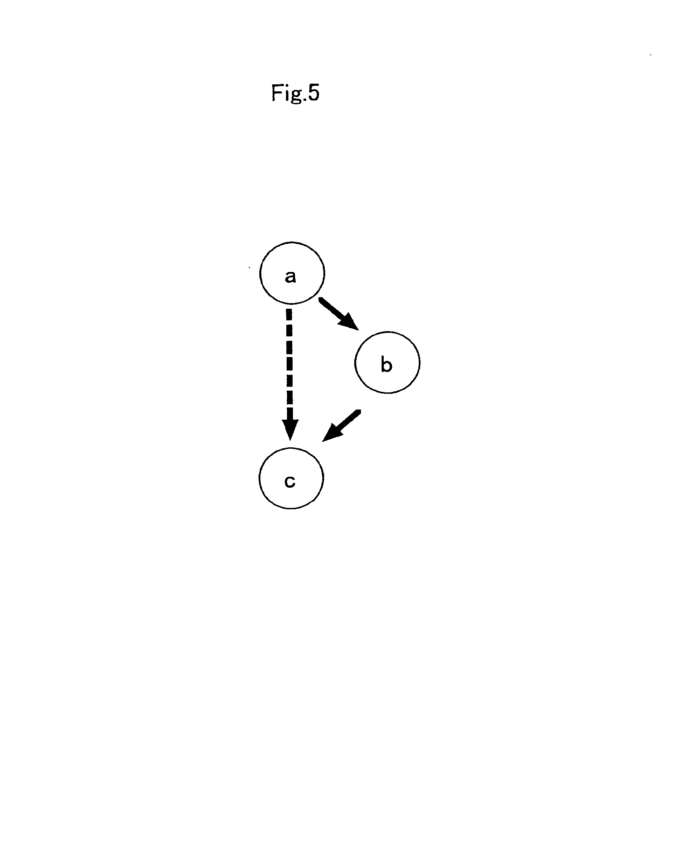

As shown in Fig. 5, a gene network wherein gene a activates gene b, and the activated gene b activates gene c, is considered. In this case, if gene a is inactivated (e.g., disrupted), gene b is not activated, and as a result gene c also is not activated. Therefore, even if gene a does not actually have the ability to activate gene c, there are cases where as a result, gene a will be falsely recognized as having the ability to indirectly activate gene c. Herein, causal relationships created in this manner due to a false recognition are referred to as "redundant causal relationships." In the present invention, such indirect edges (Fig. 5, a dotted line arrow) can be identified. In a gene regulation network in which there are indirect edges and direct edges, in the present invention, each gene is screened to determine if it imparts an indirect effect on another gene, enabling indirect edges in the network to be checked. This indirect effect depends only on the parity of the number of negative regulations involved in the edge route (Thieffry, D., and Thomas, R., Proc. Pacific Symp. Bio-computing '98, World Scientific, 77-88, 1998.)

In the present invention, a network is predicted by deleting redundant causal relationships, i.e. indirect gene regulation. Such redundant causal relationships can be found by modifying Warhsall's algorithm in the field of graph theory (Gross, J., and Yellen, J., CRC Press, 1999.). In the processing for eliminating such redundant causal relationships, a program for finding redundant causal relationships, is executed.

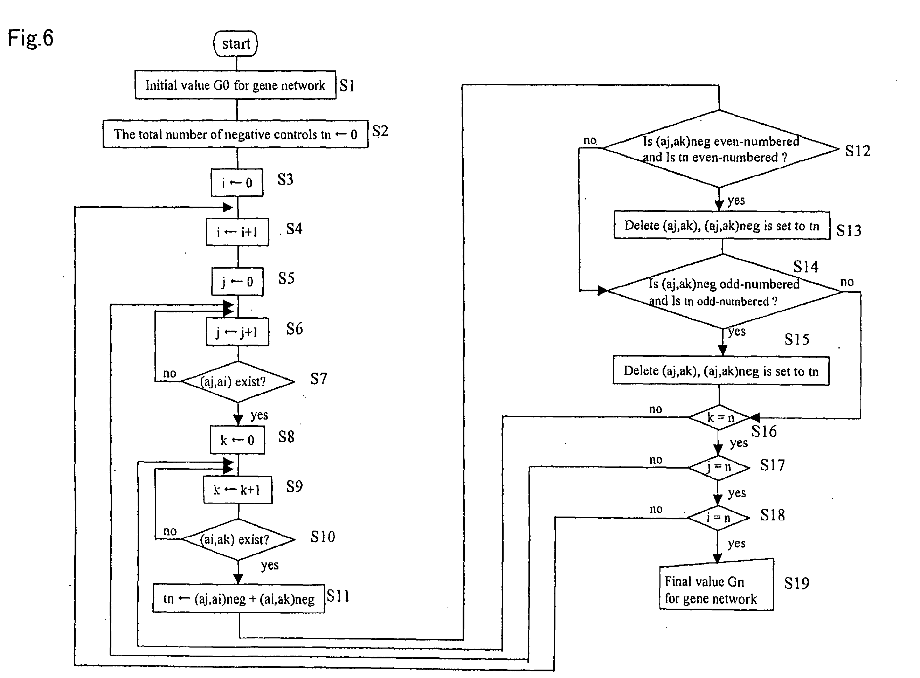

A flow chart for executing a modified Warshall algorithm is shown in Fig. 6. The steps in Fig. 6 are as follows. That is, a gene network comprising redundant causal relationships (a network from which redundant causal relationships have not been deleted) is given an initial value G0, and all genes are assigned consecutive numbers from 1 to n (the total number of genes). Next, the relationship from gene x to gene y is expressed as an element of a matrix (ax,ay), and where there is activation (positive) or inhibition (negative) relationship, (ax,ay) is set to 1 (where there is no relationship, it is set to 0). Further, where this relationship is an inhibition (negative control) relationship, a counter (ax,ay)neg possessed by each matrix element is set to 1, and otherwise it is set to 0 (S1). First, counter tn, which counts the total number of negative controls, is initialized (S2). Next, processing proceeds according to the flow chart in Fig. 6. S3 is a step wherein a process is performed to initialize index i to 0. S4 is a step in which a process is performed to set index i to i+1. S5 is a step in which a process is performed to initialize index j to 0. S6 is a step in which a process is performed to set index j to j+1. In Fig. 6, (aj,ai) refers to the relationship from gene j to gene i, and the meaning of the existence of (aj,ai) indicates that (aj,ai) is 1, and that there is a relationship from gene j to gene i. Where (aj,ai) exists (yes), in S8 a process is performed wherein index k is initialized to 0 (S7). Where (aj,ai) does not exist (no), the process of S6 is repeated.

Next, after process S8, a process is performed in S9 where index k is set to k+1, and processing proceeds to S10. "(ai,ak) exists" of S10 indicates that (ai,ak) is 1, and means that there is a relationship from gene i to gene k. In S10, where (ai,ak) exists (yes), a process is performed where tn is set to the sum of (aj,ai)neg and (ai,ak)neg (S11). Where (ai,ak) does not exist (no), the process of S9 is performed. (aj,ai)neg indicates the number of negative controls included in the pathway from gene j to gene i.

S12 is a step which compares (aj,ak) to the value of tn (S12), and where (aj, ak)neg is even-numbered and tn is even numbered (yes), a process is performed wherein (aj,ak) is set to 0, the relationship from gene j to gene k is deleted, and (aj,ak)neg is set to tn (S13). In the case where at least one of (aj,ak)neg and tn is odd-numbered (where both are not even-numbered)(no), a process is performed to compare whether (aj,ak)neg is odd-number and tn is odd-numbered (S14). In S14, where (aj,ak)neg is odd-numbered, and tn is odd-numbered (yes), a process is performed where (aj,ak) is set to 0, the relationship from gene j to gene k is deleted, and (aj,ak)neg is set to tn (S15). In S14, where at least one of (aj,ak)neg and tn is even-numbered (no), a process is performed to compare whether or not index k is n (indicating the total number of genes)(S15).

Next, in S16, a process is performed to compare whether or not index k is n. Where k=n (yes), a process is performed in S17 to compare whether or not index j is n. Where k<n (no), the process of S9 is performed.

In S17, where j=n (yes), a process is performed in S18 to compare whether or not index i is n. In the process of S17, where j<n (no), the process of S4 is performed.

In this manner, by deleting all controls between genes that are considered to be redundant in respect of all genes, gene network Gn is obtained (S19).

According to the above flow chart, the whole of a network having the possibility of a redundancy can be checked and the redundancies eliminated. The quantity of calculations of this algorithm is $O(n^3)$.

The prediction method of the present invention can be applied to not only single cell organisms but also to gene networks in cellular organisms. A gene network in a multi-cellular organism, can be predicted by further addition of the following elements to the above-described prediction method. That is, after applying the 1) detection engine for gene expression amount, (2) comparison engine, and (3) causal relationship creation engine, just as in the case of a single cell organism, a (4) redundant relationship deletion engine is applied for each cell or tissue.

2. Recording medium having a program recorded thereon

In the gene network prediction method of the present invention, it is preferable to use a computer readable recording medium on which are recorded a program for reading gene expression amounts (detected values), a program for determining a difference from the detected values, and a causal relationship creating program. These program may be recorded on separate recording media. Further, recording medium includes CD-ROM, hard disk, ROM, RAM, etc.

Brief Explanation of the Drawings

Figure 1 is a block diagram of the prediction device according to the present invention.
Figure 2 indicates a gene network.
Figure 3 indicates a relationship between expression amount of a gene and gene regulation.
Figure 4 is a directed graph indicating the prediction method of the present invention.
Figure 5 is a directed graph indicating the prediction method of the present invention.
Figure 6 is a flow chart for execution of the program to eliminate redundant causal relationships.
Figure 7 indicates a gene network predicted by the method of the present invention.
Figure 8 indicates a gene network predicted by the method of the present invention.

Explanation of Reference Numerals

101...CPU, 102...ROM, 103...RAM, 104...input unit , 105...transmit/receive part, 106...display, 107...hard disk drive, 108...CD-ROM drive, 109...CD-ROM, 110...public database

Best Mode for Carrying Out the Invention

Below, the present invention is explained in further detail by way of Examples. However, these Examples do not limit the technical scope of the present invention.

[Example 1] Prediction of a gene network

This Example is a model experiment for confirmation of the effect of the method of the present invention. A hypothetical network is prepared, and the proportion of this hypothetical gene network that can be predicted by the method of the present invention is confirmed.

The number of genes, N, was computationally established as N=10, 20, 50 or 100, and the maximum indegrees, k was established as 2, 4 or 8. Maximum indegrees refers to the maximum number of genes from which a particular gene is controlled. Based on these settings, and using a random number table, 100 patterns of networks T constituted by directed graphs, were randomly simulated for the groups of genes according to the respective numerical settings. It should be noted that each network was programmed to include cyclic regulation networks but not include self-regulation networks.

Parameters and regulation type for the model formula (in formula (I), values Ra, Wab and λa), i.e. parameters for each gene regarding whether they were regulated without any relationship to each other gene, or were regulated cooperatively, were randomly determined.

Network Model

$$\frac{dv^a}{dt} = R_a g \left( \sum_b W^{ab} v^b + h^a \right) - \lambda_a v^a \qquad \text{(I)}$$

(formula I, $R_a$ represents the rate determining the maximum amount produced from gene a in infinitesimal time interval, dt, g represents a sigmoid function ,$W^{ab}$ represents the weight of influence from $v^b$, $v^b$ indicates the gene expression level of gene b, $h^a$ represents the influence of general transcription factors on gene a, $\lambda_a$ represents the rate of degradation of the gene product of gene a, and $v^a$ represents the gene expression level of gene a)

Expression amounts of each gene in the wild type gene network were simulated by calculating and stabilizing with 1000 simulation steps with the model formula, and expression amounts of the mutant gene network (wherein the function of a specific gene in the network has been disrupted) were simulated in the same way as with the wild type gene network after setting parameter Ra of the corresponding gene to zero. Networks with each gene independently deleted (single deletion networks) were respectively prepared for all genes present in the network, and simulated.

The extent to which the networks predicted by the method of the present invention differed from target networks was determined as a rate relative to networks (target networks) simulated as described above.

The points of difference between the predicted network and the target network were evaluated by two standards (sensitivity and specificity). "Sensitivity" is defined as the proportion of the number correctly predicted edges relative to the number of edges in the target network. This sensitivity indicates the percentage (%) of the number of edges included in the target network that are included in the predicted network, relative to the total number of edges in the simulated target network. For example, if there are 10 edges in the target network, and the number of edges present in the predicted network and in the target network is 8, then the sensitivity is 80%.

Further, specificity refers to the percentage (%) of correctly predicted edges relative to the total number of edges present in the predicted network.

This specificity indicates how many edges among the edges in the predicted network match with edges in the target network. According to the above described example, if among 8 edges present in the predicted network, 7 edges matched with edges present in the target network, then the specificity becomes 87.5%.

Experimental results are shown in Table 2. Because 100 types of target network were created when simulated assuming 10 genes, the prediction operation is performed 100 times (this is the same in the cases of 20, 50 and 100 genes). Therefore, data for sensitivity, specificity, etc. (Table 2) indicates the average value of 100 prediction operations.

| Table 2 | | | | | | |
|---|---|---|---|---|---|---|
| N | k | total number of simulated edges | total number of predicted edges | number of matching edges | sensitivity | specificity |
| 10 | 2 | 15.2 (1.6) | 8.9 (3.0) | 8.1 (2.9) | 53.1% | 90.4% |
| 20 | 2 | 30.5 (0.6) | 20.6 (4.9) | 18.6 (4.7) | 61.1% | 90.6% |
| 50 | 2 | 75.5 (0.7) | 60.7 (8.7) | 54.4 (7.9) | 72.1% | 89.8% |
| 100 | 2 | 150.4 (0.6) | 133.9 (10.4) | 119.2(9.5) | 79.2% | 89.1% |
| 20 | 4 | 80.0 (0.0) | 20.3 (8.9) | 17.0 (7.4) | 21.3% | 84.1% |
| 20 | 8 | 117.1 (11.8) | 15.1 (8.6) | 9.6 (6.1) | 8.1% | 61.3% |

Each measured value is the average value for the simulated 100 target networks, and standard error is shown in parenthesis (). The total number of edges refers to the average number ± standard error of the number of arrows (arrows indicating activation or inhibition) joining two nodes in the network.

As is clear from Table 2, the average specificity value is always higher than the average sensitivity value, and further, sensitivity increased in proportion to the network size, N.

The average specificity value was unrelated to N, and was approximately 90% at in-degree k=2. That is, if a target network is considered where the gene number is 20, the average number of edges drawn in the target network is 30.5, and the number of edges in the predicted network is 20.6. Among these 20.6 edges, the number of edges matching the edges in the target network was 18.6.

Where specificity is high even where sensitivity is low, this means that even if the number of edges in the predicted network is not compatible with the number of edges in the target network, the edges that have been drawn have a high probability of matching edges in the target network. Therefore, the method of the present invention enables prediction of a network at a very high proportion.

It should be noted that the average values for sensitivity and specificity decrease in proportion to an increase in k.

[Example 2] Comparison of continuous-value data and binary data

One main characteristic of the method of the present invention is the application of a continuous value for expression data. To clarify the advantage of using continuous-value expression data, the present inventor carried out a comparison of the sensitivity and specificity between a case where continuous-value data is used and a case where binary data is used. In the binary case, continuous expression data was corrected to binary data in accordance with a threshold value. This threshold value was established as an intermediate value between minimum expression level $x_{min}$ and maximum expression level $x_{max}$. However, minimum expression level $x_{min}$ was defined as 0 (zero) (1/2 of $x_{max}$ was taken as a threshold value). Gene number, detection and calculation of expression amounts, and prediction processing were the same as that in Example 1.

Results are shown in Table 3. Respective measured values are average values of the simulated 100 target networks. Prediction results when continuous-values were used substantially exceeded prediction values when binary data was used in respect of both sensitivity and specificity.

| Table 3 | | | | | |
|---|---|---|---|---|---|
| N | k | Continuous-value Data | | Binary data | |
| | | Sensitivity | Specificity | Sensitivity | Specificity |
| 10 | 2 | 53.1% | 90.4% | 20.3 % | 58.6 % |
| 20 | 2 | 61.1% | 90.6% | 20.9 % | 61.7% |
| 50 | 2 | 72.1% | 89.8% | 22.2 % | 63.1 % |
| 100 | 2 | 79.2% | 89.1% | 22.9 % | 65.3 % |
| 20 | 4 | 21.3% | 84.1% | 9.7 % | 60.1 % |
| 20 | 8 | 8.1% | 61.3% | 6.4 % | 47.3 % |

[Example 3] Comparison with conventional methods

Because conventionally known gene network prediction methods are designed to analyze binary data, they cannot be applied to continuous-value data.

In this Example, a prediction method using the method of the present invention and a prediction method according to a conventional method were compared. With the conventional method, original data obtained in the same manner as in Example (1), and data corrected into binary data as in Example 2 was used, and with the method of the present invention, the above original data was used as is. Gene number, detection and calculation of expression amounts, and prediction processing were the same as in Example 1.

As a result, the method of the present invention was shown to be superior when compared with the conventional method (Table 4).

Table 4:

| Results of prediction when the method of the present invention and a conventional method were used | | | | | |
|---|---|---|---|---|---|
| N | K | method of the present invention | | conventional method | |
| | | sensitivity | specificity | sensitivity | specificity |
| 10 | 2 | 53.1% | 90.4% | 7.1% | 12.5% |
| 20 | 2 | 61.1% | 90.6% | 7.9% | 8.4% |
| 50 | 2 | 72.1% | 89.8% | 3.3% | 7.7% |
| 100 | 2 | 79.2% | 89.1% | * | * |
| 20 | 4 | 21.3% | 84.1% | 9.2% | 23.4% |
| 20 | 8 | 8.1% | 61.3% | 8.2% | 34.8% |

Each measured value is an average value of values obtained from 100 types of target network that were simulated. The symbol, "*", represents that it was not possible to perform calculations.

Where the number of genes was 20 (N=20), and k=8, the conventional method had sensitivity that was slightly higher than the method of the present invention, however, there was no statistically significant difference between these two methods ($p<0.05$). This result means that even if a conventional method can predict edges from artificial gene expression data formed by using a Boolean network model, it would be ill-suited to actual gene expression data.

Because the amount of calculations in the method of the present invention is $O(n^3)$, a gene regulation network could be predicted even where N=100 and k=2.

[Example 4] Gene network prediction using expression amounts obtained from a DNA microarray

In this example, a gene network was predicted using gene expression data obtained by measuring expression amounts of genes (approx. 250 types) from, as an actual living material, yeast (Saccharomyces cerevisiae), using a DNA microarray.

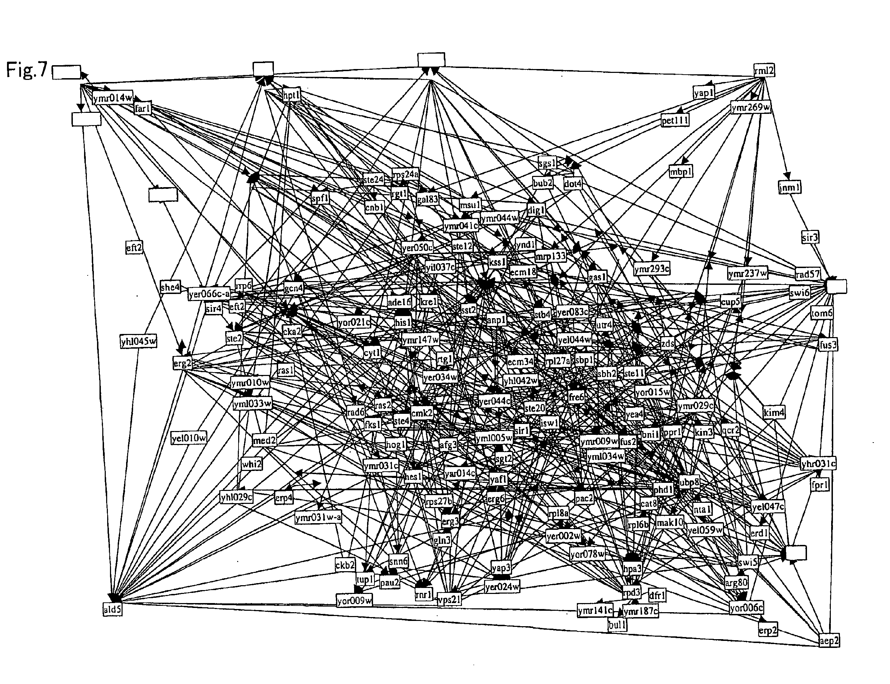

The gene expression data that was used was data published in a thesis by Hughes et al. (Hughes, T. R. et al., Cell, 102:109-126, 2000.), where ratios of gene expression levels of wild types and mutant types were measured using a microarray. This data is called a "compendium", apart from gene expression level ratios, gene expression level ratios are accompanied by values, called p-values, indicating the significance of the difference arising between the expression level of the mutant type relative to the wild type. The lower this value, the more significant the difference in gene expression level relative to the wild type. In this Example, there was deemed to be a difference in expression level where p-value was 0.01 or less, otherwise, it was deemed there was no difference in comparison with the wild type. From among this data, single gene deletion type gene expression data only was retrieved. Further, only expression data of deleted genes was selected, and expression data for approximately 250 genes in relation to approximately 250 gene deletion-types was retrieved and the method of the present invention was applied. As a result, the gene network shown in Fig. 7 could be predicted.

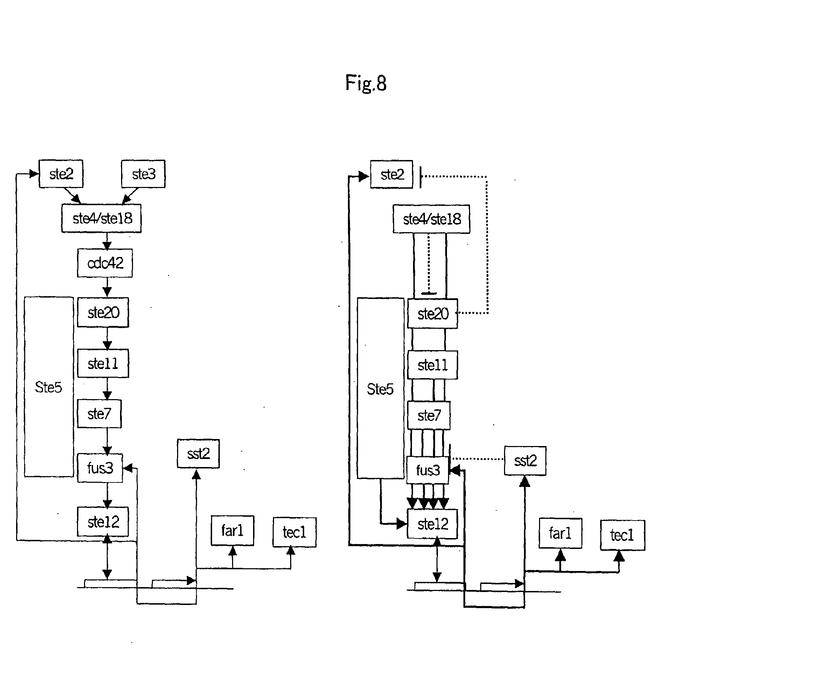

Further, prediction according to the method of the present invention was performed in respect of a network known in the literature. Results are shown in Figure 8. The left panel of Fig. 8 shows the network as known in the literature (Roberts, C. J. et al., Science, 287(5454): 873-880, 2000.; Oehlen, L. J. et al., Mol. Cell. Biol., 16(6): 2830-2837, 1996.; Dietzel, C. and Kurjan, J., Mol. Cell. Biol., 7(12): 4169-4177, 1987.; Errede, B. and Ammerer, G., Genes and Dev., 3(9):1349-1361, 1989..; Oehlen, L. and Cross, F. R., FEBS Lett., 429(1): 83-88, 1998.) and the right panel is the network predicted by the prediction method of the present invention. In respect of genes upstream of ste12, which is a transcription factor, the above publication specifies a phosphorylation relay relationship from ste2 to ste12. According to the method of the present invention, because the microarray expression data measures mRNA expression level, and ste12 is self-controlled, the relationship from genes upstream from ste12 to ste12 was predicted. In connection with the genes controlled by ste12, according to the above publication, there are fus3, tecl, far1, ste2 and sst2. According to the method of the present invention, from the fact that microarray expression data measures mRNA expression level, relationships to fus3, tecl, far1, ste2 and sst2, matching with the publication, were predicted.

Therefore, the method of the present invention was able to predict a reasonable gene network in comparison to the known gene network as a network predicted from microarray data.

All publications, patents and patent applications cited herein, are incorporated herein in their entirety.

Industrial Applicability

The present invention provides a method of predicting a gene network. A network obtained by the method of the present invention is useful in, for example, development of medicaments (drug design, etc.).

**Claims**

1.  A method of predicting a gene network which comprises detecting, when an expression amount of a single gene among a plurality of genes is expressed under 2 conditions, the respective expression amounts of another gene; determining a difference between the detected values; and deriving a causal relationship between the single gene and the other gene using the difference as an indicator.

2.  A method of predicting a gene network which comprises respectively detecting, when a single gene among a plurality of genes has been mutated, an expression level of another gene, and when the single gene has not been mutated, an expression level of the other gene; determining the difference between the obtained detected values; and deriving a causal relationship between the single gene and the other gene using the difference as an indicator.

3.  A prediction method according to claim 1 or 2, which further comprises verifying the presence or absence of a contradiction in an obtained causal relationship.

4.  A prediction method according to claim 1 or 2, wherein the expression level of a gene is a level of phosphorylation of the gene.

5.  A prediction method according to claim 2 wherein the gene mutation is a gene disruption.

6.  A prediction method according to claim 1 or 2 wherein the gene network is a gene network in a single cell organism.

7.  A prediction method according to claim 1 or 2 wherein the gene network is a gene network in a multi-cellular organism.

8.  A gene network prediction system comprising a means for detecting, when an expression amount of a single gene among a plurality of genes is expressed under 2 conditions, the respective expression amounts of another gene;

a means for determining a difference between the detected values; and a means for deriving a causal relationship between the single gene and the other gene using the difference as an indicator.

9. A prediction system according to claim 8, which further comprises verifying the presence or absence of a contradiction in an obtained causal relationship.

10. A computer readable recording medium on which there is recorded a program causing a computer to function as a gene network prediction system, which comprises a means for detecting, when a single gene among a plurality of genes has been mutated, an expression level of another gene, and when the single gene has not been mutated, an expression level of the other gene; a means for determining a difference between the detected values; a means for deriving a causal relationship between the single gene and the other gene using the difference as an indicator; and a means for verifying the presence or absence of a contradiction in an obtained causal relationship.

Fig.1

Fig.2

Fig.3A

|  | $a_0$ | $a_1$ | $a_2$ | $a_3$ |
|---|---|---|---|---|
| $a_0$ |  |  | + | + |
| $a_1$ |  |  | + |  |
| $a_2$ |  |  |  |  |
| $a_3$ |  |  |  | − |

Fig.3B

|  | $x_0$ | $x_1$ | $x_2$ | $x_3$ |
|---|---|---|---|---|
| wt | 3.750 | 3.750 | 8.939 | 0.078 |
| $a_0^-$ | − | 3.750 | 8.769 | 0.011 |
| $a_1^-$ | 3.750 | − | 8.769 | 0.086 |
| $a_2^-$ | 3.750 | 3.750 | − | 5.476 |
| $a_3^-$ | 3.750 | 3.750 | 8.939 | − |

Fig.4A

Fig.4B

Fig.4C

Fig.4D

Fig.5

Fig.6

start

Initial value G0 for gene network | S1

The total number of negative controls tn ← 0 | S2

i ← 0    S3

i ← i+1    S4

j ← 0    S5

j ← j+1    S6

(aj,ai) exist?    S7
no / yes

k ← 0    S8

k ← k+1    S9

(ai,ak) exist?    S10
no / yes

tn ← (aj,ai)neg + (ai,ak)neg   S11

Is (aj,ak)neg even-numbered and Is tn even-numbered ?    S12
no / yes

Delete (aj,ak), (aj,ak)neg is set to tn   S13

Is (aj,ak)neg odd-numbered and Is tn odd-numbered ?   S14
no / yes

Delete (aj,ak), (aj,ak)neg is set to tn   S15

k = n    S16
no / yes

j = n    S17
no / yes

i = n    S18
no / yes

Final value Gn for gene network   S19

16

EP 1 342 782 A1

Fig.7

Fig.8

# INTERNATIONAL SEARCH REPORT

| International application No. |
| --- |
| PCT/JP01/09909 |

A. CLASSIFICATION OF SUBJECT MATTER
    Int.Cl⁷   C12N15/00, G06F19/00, C12M1/00

According to International Patent Classification (IPC) or to both national classification and IPC

B. FIELDS SEARCHED

Minimum documentation searched (classification system followed by classification symbols)
    Int.Cl⁷   C12N15/00, G06F19/00, C12M1/00

Documentation searched other than minimum documentation to the extent that such documents are included in the fields searched

Electronic data base consulted during the international search (name of data base and, where practicable, search terms used)
    WPIDS/BIOSIS/BIOTECHABS/MEDLINE (STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | AKUTSU, T. et al., "Inferring qualitative relations in genetic networks and metabolic pathways", Bioinformatics, August, 2000, Vol.16, No.8, pages 727 to 734 | 1,2,4-8,10<br>3,9 |
| Y<br>A | Gross, J. et al., "Graph theory and its applications", CRC Press, (1999) | 3,9<br>1,2,4-8,10 |
| P,X | KYODA, K. et al., "A gene network inference method from continuous-value gene expression data of wild-type and mutants", Genome Informatics, (2000), Vol.11, pages 196 to 204 | 1-10 |
| A | DeRisi, J. et al., "Exploring the metabolic and genetic control of gene expression on a genomic scale", Science, (1997), Vol.278, pages 680 to 686 | 1-10 |
| A | Brown, M. et al., "Knowledge-based analysis of microarray gene expression data by using support vector machines", Proc. Natl. Acad. Sci. USA, January, 2000, Vol.97, No.1, pages 262 to 267 | 1-10 |

☐ Further documents are listed in the continuation of Box C.   ☐ See patent family annex.

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |
| Date of the actual completion of the international search<br>    05 February, 2002 (05.02.02) | Date of mailing of the international search report<br>    19 February, 2002 (19.02.02) |
| Name and mailing address of the ISA/<br>    Japanese Patent Office | Authorized officer |
| Facsimile No. | Telephone No. |

Form PCT/ISA/210 (second sheet) (July 1992)